(19) Europäisches Patentamt
European Patent Office
Office européen des brevets

(11) Publication number : **0 408 273 B1**

(12) **EUROPEAN PATENT SPECIFICATION**

(45) Date of publication of patent specification :
**19.01.94 Bulletin 94/03**

(51) Int. Cl.$^5$ : **A61K 31/675,** A61K 9/20

(21) Application number : **90307449.0**

(22) Date of filing : **09.07.90**

(54) **Fosinopril tablet formulations.**

(30) Priority : **10.07.89 US 377683**

(43) Date of publication of application :
**16.01.91 Bulletin 91/03**

(45) Publication of the grant of the patent :
**19.01.94 Bulletin 94/03**

(84) Designated Contracting States :
**AT BE CH DE DK ES FR GB GR IT LI LU NL SE**

(56) References cited :
**EP-A- 0 264 888**
**EP-A- 0 288 907**
**US-A- 4 217 347**
**US-A- 4 384 123**
**LEXIKON DER HILFSSTOFFE FÜR PHARMAZIE, KOSMETIK UND ANGRENZENDE GEBIETE, vol. II, 1989, Editio Cantor Aulendorf; H.P.FIEDLER, p. 858**

(73) Proprietor : **E.R. SQUIBB & SONS, INC.**
**Lawrenceville-Princeton Road**
**Princeton New Jersey 08543-4000 (US)**

(72) Inventor : **Jerzewski, Robert L.**
**P.O.Box 2044**
**East Millstone, NJ (US)**
Inventor : **Wong, Thomas M.**
**18 Parkside Drive**
**North Brunswick, NJ (US)**
Inventor : **Gryziewicz, Lewis J.**
**918 Birchwood Ct.**
**North Brunswick, NJ (US)**
Inventor : **Jain, Nemichand B.**
**24 Hillside Avenue**
**Monmouth Junction, NJ (US)**
Inventor : **Thakur, Ajit B.**
**20 Lawry Court**
**East Brunswick, NJ (US)**

(74) Representative : **Thomas, Roger Tamlyn et al**
**D. Young & Co. 21 New Fetter Lane**
**London EC4A 1DA (GB)**

**EP 0 408 273 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The sodium salt of fosinopril, (4S)-4-cyclohexyl-1-[[[(RS)-1-hydroxy-2-methylpropoxy](4-phenylbutyl)phosphinyl]acetyl]-L-proline, propionate (ester) sodium salt, is currently undergoing clinical evaluation as an antihypertensive agent.

Fosinopril, its ability to inhibit the angiotensin converting enzyme and thus lower blood pressure in humans, and formulations of fosinopril including combinations with various diuretics are described in United States Patents US-A-4,337,201 and US-A-4,384,123.

The present invention is concerned with the use of particular lubricants in tablets containing fosinopril sodium. European Patent Application EP-A-288907 discloses an Angiotensin Converting Enzyme Inhibitor tablet formulation suitable for oral administration comprising gelatin as a binder, corn starch as a disintegrant, microcrystalline cellulose or lactose as a filler and magnesium stearate as a lubricant. Lexikon der Hilfstoffe fuer Pharmazie, Kosmetik und angegrenzde Gebiete, H.P. Fiedler, Band II (L-Z), 1989, Editio Cantor Aulendorf, page 858, "Natriumstearylfumarat" indicates that sodium stearyl fumarate is an alternative to magnesium stearate.

This invention is directed to the discovery that the shelf life and stability of fosinopril sodium formulated as tablets is increased when the lubricant employed is sodium stearyl fumarate or hydrogenated vegetable oil as compared to tablets employing magnesium stearate as the lubricant. In addition to the fosinopril sodium, the tablet formulation can also include a diuretic, preferably chlorthalidone, a filler, a disintegrant, a binder, a lubricant, and other commonly employed pharmaceutically acceptable agents. The tablet can include a color agent or the tablets can be color film coated. The tablets can be prepared in a variety of shapes and can be scored for the convenience of the user.

More specifically the invention provides a stable tablet comprising, on a weight percentage basis, from 1% to 25% fosinopril sodium, from 0% to 25% of a diuretic, from 30% to 90% of a filler, from 2% to 10% of a disintegrant, from 1% to 5% of a binder, or from 5% to 15% of a single agent which is both binder and disintegrant, and from 0.3% to 4% of a lubricant selected from sodium stearyl fumarate and hydrogenated vegetable oil.

Fosinopril sodium having the chemical formula

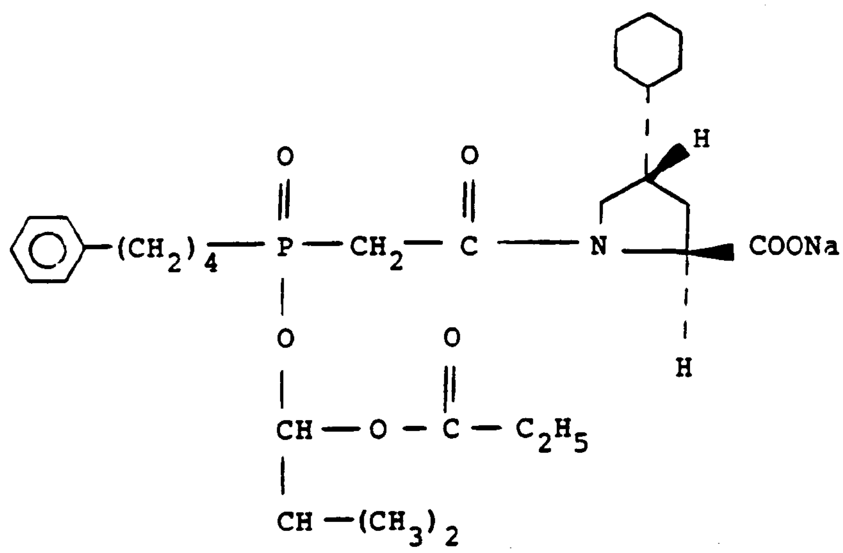

is an angiotensin converting enzyme inhibitor currently being clinically evaluated as an anti-hypertensive agent.

Fosinopril sodium bulk material has a relatively low bulk density, exhibits poor flow characteristics, and adheres to metal surfaces during tableting. Previously, fosinopril sodium was tableted by a wet granulation process in which the tablet binder material was added to a solvent to form a solution of about 20% on a weight to weight basis.

The fosinopril sodium and a portion of the tablet filler and disintegrant were then added. The mix was granulated and dried to less than 1% by weight of volatiles. The remainder of the tablet excipients were added and the final blend was obtained by lubrication with magnesium stearate. It was found that tablets produced from this blend were moisture sensitive and only marginally stable. In order to have a useful shelf life these tablets required the use of a protective package.

This invention is based on the discovery that by employing sodium stearyl fumarate or hydrogenated vegetable oil as the lubricant during the tableting of fosinopril sodium (instead of magnesium stearate), tablets having improved stability are obtained. The tablets thus prepared are significantly less moisture sensitive and have a useful shelf life without the need for protective packaging. Sodium stearyl fumarate is the preferred lubricant since hydrogenated vegetable oil can cause processing problems of sticking to the punch tips during long tableting runs.

As discussed in United States Patents US-A-4,337,201 and US-A-4,384,123, various diuretics can be combined with fosinopril sodium for the treatment of hypertension. Suitable diuretics include chlorthalidone, the thiazide diuretics such as chlorothiazide, hydrochlorothiazide, flumethiazide, bendroflumethiazide, etc., ethacrynic acid, ticrynafen, furosemide, musolimine, bumetanide, triameterene, amiloride, spironolactone, and salts thereof with chlorthalidone and hydrochlorothiazide being preferred.

Tablets prepared according to this invention generally contain from 5 mg. to 50 mg. of fosinopril sodium as the sole active agent or from 10 mg. to 75 mg. of a combination of fosinopril sodium and diuretic in a ratio of from 1:5 to 5:1 on a weight basis.

As discussed above, in addition to the actives, fosinopril sodium and the optional diuretic, and the lubricant, tablets prepared according to this invention will include excipients such as a filler, a disintegrant, and a binder. The preferred filler is lactose or lactose and microcrystalline cellulose. The preferred disintegrants are selected from sodium carboxymethyl starch, cross linked sodium carboxymethyl starch, crospovidone, i.e., 1-ethenyl-2-pyrrolidinone homopolymer, cross linked sodium carboxymethylcellulose (AcDiSol® or Croscarmellose Sodium®), sodium starch glycolate, and mixtures thereof. The preferred binders are selected from povidone, i.e., 2-pyrrolidinone, 1-ethenyl-, homopolymer, hydroxypropyl cellulose, and mixtures thereof. Alternatively, a single agent such as pregelatinzed starch can be employed as both disintegrant and binder. Other ingredients commonly employed in tableting pharmaceutical products can also be included such as coloring agents.

On a weight percentage basis, the above ingredients will preferably be present in the final tablets as follows:

| Ingredient | Preferred percentage by weight |
|---|---|
| Fosinopril sodium | 1 to 25 |
| diuretic, (preferably chlorthalidone or hydrochlorothiazide) | from 0 up to 25 |
| filler (preferably lactose or lactose combined with micro-crystalline cellulose) | 30 to 90 |
| disintegrant (preferably sodium carboxymethyl starch and its cross linkaged form and/or crospovidone and/or crosslinked sodium carboxy-methylcellulose and/or sodium starch glycolate) | 2 to 10 |
| binder (preferably povidone and/or hydroxy-propyl cellulose) | 1 to 5 |
| combined binder and disintegrant (preferably pregelatinized starch) | 5 to 15 |
| lubricant (preferably sodium stearyl fumarate) | 0.3 to 4 |

The fosinopril sodium tablets of this invention can be prepared by conventional tablet forming techniques such as, for example, wet granulation and dry granulation. In the wet granulation process, the active ingredient or ingredients are mixed with portions of the filler and disintegrant. This blend is then wet granulated with a solution of the binder in a solvent. The solution will preferably be from 5% to 20% by weight of solvent and the preferred solvents include ethanol, isopropanol, and water. The resultant wet granulation is then dried and milled. The dried granulation is then mixed with the balance of the filler and disintegrant. The resulting blend is mixed with sodium stearyl fumarate, which is preferred, or hydrogenated vegetable oil to produce the final mix which is then compressed into tablets.

In the dry granulation process, the active ingredient or ingredients and the filler, disintegrant and binder

are blended in a mixer (planetary or high shear) for several minutes. The blend is then milled and mixed with sodium stearyl fumarate, which is preferred, or hydrogenated vegetable oil to produce the final mix which is then compressed into tablets.

The wet granulation process employing water as the solvent and the dry granulation process have the added advantage of avoiding the use of an organic solvent. This results in a cost savings as well as a safer process needing fewer environmental controls.

Example 1

Tablets were prepared containing:

| Ingredient | Weight(mg/) |
|---|---|
| Fosinopril sodium | 40.0 |
| Chlorthalidone | 15.0 |
| Lactose | 318.00 |
| Povidone | 9.0 |
| Crospovidone | 14.0 |
| Sodium stearyl fumarate | 4.0 |
| Water | q.s. |
| Tablet weight | 400 |

100,000 tablets of the above formulation were prepared as follows. A blend was prepared of fosinopril sodium (4000 g), chlorthalidone (1500 g), lactose (17,900 g), and crospovidone (700 g) in a suitable mixer such as planetary mixer or high shear mixer for 5 to 10 minutes. Povidone (900 g) was dissolved in water (7 liters) and the above blend was wet granulated with the entire povidone solution. The wet granulation was dried at 45 - 70° C. in a suitable dryer such as a tray drying oven or a fluid bed dryer until the volatile content of the wet granulation was less than 3% by weight. The dried granulation was passed through a hammer mill fitted with 0.03 - 0.07 inch screen operating at medium to fast speed, knives forward. The screened granulation was then mixed with lactose (13,900 g) and crospovidone (700 g) in the mixer (planetary or high shear) for 5 to 10 minutes. Sodium stearyl fumarate (400 g) was then added to the above blend and mixing was continued for 1 to 3 minutes. The final blend was then compressed into 400 mg tablets using a rotary tablet press.

Examples 2 to 5

Following the procedure of Example 1, the following tablets were obtained.

| Ingredient | Weight (mg) | | | |
|---|---|---|---|---|
| | 2 | 3 | 4 | 5 |
| Fosinopril sodium | 20.0 | 10.0 | 5.0 | 5.0 |
| Chlorthalidone | 15.0 | 15.0 | 25.0 | 5.0 |
| Lactose | 244.75 | 161.0 | 62.5 | 82.5 |
| Povidone | 6.75 | 4.5 | 3.0 | 3.0 |
| Crospovidone | 10.50 | 7.5 | 3.5 | 3.5 |
| Sodium stearyl fumarate | 3.0 | 2.0 | 1.0 | 1.0 |
| Water | q.s. | q.s. | q.s. | q.s. |
| Tablet weight | 300 | 200 | 100 | 100 |

Examples 6 to 9

Following the procedure of Examples 1 to 5, the following tablets were obtained.

| Ingredient | Weight (mg) | | | |
|---|---|---|---|---|
| | 6 | 7 | 8 | 9 |
| Fosinopril sodium | 20.0 | 10.0 | 20.0 | 10.0 |
| Hydrochlorthiazide | 12.5 | 12.5 | 12.5 | 12.5 |
| Lactose | 237.5 | 157.5 | 112.5 | 123.5 |
| Avicel® (Microcrystalline cellulose | -- | -- | 40.0 | 40.0 |
| Povidone | 6.0 | 4.0 | 4.0 | 4.0 |
| Croscarmellose Sodium® (cross linked sodium carboxymethylcellulose) | 15.0 | 10.0 | -- | -- |
| Sodium starch glycolate | -- | -- | 7.0 | 7.0 |
| Sodium stearyl fumarate | 9.0 | 6.0 | 4.0 | 3.0 |
| Water | q.s. | q.s. | q.s. | q.s. |
| Tablet weight | 300 | 200 | 200 | 200 |

Examples 10 and 11

The following tablets were prepared by a modification of the procedures of Examples 1 to 9.

| Ingredients | Weight (mg) | |
|---|---|---|
| | 10 | 11 |
| Fosinopril sodium | 20.0 | 10.0 |
| Hydrochlorothiazide | 12.5 | 12.5 |
| Lactose | 107.5 | 118.5 |
| Avicel® (micro-crystalline cellulose) | 40.00 | 40.00 |
| Pregelatinized starch | 16.0 | 16.0 |
| Sodium stearyl fumarate | 4.0 | 3.0 |
| Water | q.s. | q.s. |
| Tablet weight | 200 | 200 |

In the preparation of the tablets of Examples 10 and 11, a portion of the pregelatinized starch was added before the wet granulation step and the remainder was added to the dried granulation.

Example 12

Tablets were prepared containing the following:

| Ingredient | Weight(mg) |
|---|---|
| Fosinopril sodium | 5.0 |
| Lactose | 139.5 |
| Avicel® (microcrystalline cellulose) | 40.0 |
| Crospovidone | 7.0 |
| Povidone | 4.5 |
| Sodium stearyl fumarate | 4.0 |
| Alcohol (used for processing, not present in tablet) | q.s |
| Tablet Weight | 200 |

200,000 tablets of the above formulation were prepared as follows. Fosinopril sodium (1,000 g), lactose (16,500 g), Avicel (1,000 g) and crospovidone (700 g) were mixed in a suitable mixer (planetary or high shear) for 5 to 10 minutes. Povidone (800 g) was dissolved in denatured alcohol (4 liters) and blended with the above mixture forming a wet granulate. This wet granulate was dried at 45 - 70° C. in suitable dryer such as a tray drying oven or a fluid bed dryer until the volatile content of the wet granulation was less than 3% by weight. The dried granulation was passed through a hammer mill fitted with a 0.76 mm - 1.75 mm (0.03 - 0.07 inch) screen operating at medium to fast speed, knives forward. The screened granulation was mixed with lactose (12,100 g), Avicel® (7,000 g) and crospovidone (700 g) in a suitable mixer (planetary or high shear) for 5 to 10 minutes. Sodium stearyl fumarate was added to the above blend and mixed for 1 to 3 minutes in the same mixer. This final blend was then compressed into 200 mg tablets using a rotary tablet press.

Examples 13 and 14

Following the procedure of Example 12, the following tablets were obtained.

| Ingredient | Weight (mg) 13 | Weight (mg) 14 |
|---|---|---|
| Fosinopril sodium | 10.0 | 20.0 |
| Lactose | 134.5 | 124.5 |
| Avicel® (microcrystalline cellulose) | 40.0 | 40.0 |
| Crospovidone | 7.0 | 7.0 |
| Povidone | 4.5 | 4.5 |
| Sodium stearyl fumarate | 4.0 | 4.0 |
| Alcohol (used for processing, not present in tablet) | q.s | q.s |
| Tablet Weight | 200 | 200 |

Example 15

Tablets were prepared containing the following:

| Ingredient | Weight(mg ) |
|---|---|
| Fosinopril sodium | 5.0 |
| Lactose | 143 |
| Avicel® (microcrystalline cellulose) | 40.0 |
| Crospovidone | 7.0 |
| Povidone | 4.0 |
| Sodium stearyl fumarate | 1.0 |
| Tablet weight | 200 mg. |

200,000 tablets of the above formulation were prepared as follows. Fosinopril sodium (1,000 g), lactose (28,600 g), Avicel® (8,000 g), crospovidone (1400 g) and povidone (800 g) were mixed in a suitable mixer (planetary or high shear) for 5 to 10 minutes. The blend was then passed through a hammer mill equipped with a 0.04 - 0.08 inch size round hole screen operating at medium speed, knives forward. Sodium stearyl fumarate (200 g) was added to the above blend and mixed for 1 to 3 minutes in the same mixer. This final blend was then compressed into 200 mg. tablets using a rotary tablet press.

Following the procedures of Examples 1 to 5 and 12 to 15, similar tablets were prepared substituting hydrogenated vegetable oil for the sodium stearyl fumarate.

Example 16

Following the procedure of Example 12 fosinopril sodium tablets were prepared utilizing sodium stearyl fumarate, hydrogenated vegetable oil, and magnesium fumarate as the lubricant. The initial amount of fosinopril

sodium was measured and similar measurements were made after 10 days and 25 days storage under varying conditions.

| Storage condition | Weight Of Fosinopril Sodium | | |
|---|---|---|---|
| | Sodium stearyl fumarate formulation | Hydrogenated vegetable oil formulation | Magnesium stearate formulation |
| Initial | 4.99 mg | 4.86 mg | 4.95 mg |
| 10 days at 75°C. in closed containers | 4.36 mg | 4.71 mg | 3.44 mg |
| 10 days at 50°C, 75% relative humidity in open containers | 4.87 mg | 4.60 mg | 3.44 mg |
| 10 days at 60°C, 75% relative humidity in open containers | 4.36 mg | 3.92 mg | 0.90 mg |
| 25 days at 75°C, in closed containers | 4.25 mg | 4.37 mg | 3.39 mg |
| 25 days at 50°C, 75% relative humidity in open containers | 4.69 mg | 4.31 mg. | 1.83 mg |

## Claims

**Claims for the following Contracting States : AT, BE, CH, DE, DK, FR, GB, IT, LI, LU, NL, SE**

1. A stable tablet comprising, on a weight percentage basis, from 1% to 25% fosinopril sodium, from 0% to 25% of a diuretic, from 30% to 90% of a filler, from 2% to 10% of a disintegrant, from 1% to 5% of a binder, or from 5% to 15 %of a single agent which is both binder and disintegrant, and from 0.3% to 4% of a lubricant selected from sodium stearyl fumarate and hydrogenated vegetable oil.

2. The tablet of Claim 1 wherein said lubricant is sodium stearyl fumarate.

3. The tablet of Claim 2 wherein said filler is lactose or a blend of lactose and microcrystalline cellulose, said

disintegrant is selected from sodium carboxymethyl starch, cross linked sodium carboxymethyl starch, crospovidone, cross linked sodium carboxymethylcellulose, sodium starch glycolate, and mixtures thereof, said binder is selected from povidone, hydroxypropyl cellulose, and mixtures thereof, and said single agent which is both binder and disintegrant is pregelatinized starch.

**4.** The tablet of Claim 3 containing from 1% to 25% by weight of chlorthalidone.

**5.** The tablet of Claim 4 wherein said filler is lactose, said disintegrant is crospovidone, and said binder is povidone.

**6.** The tablet of Claim 5 containing on a weight percentage basis 10% fosinopril sodium, 3.75% chlorthalidone, 79.5% lactose, 2.25% povidone, 3.5% crospovidone, and 1% sodium stearyl fumarate.

**7.** The tablet of Claim 5 containing, on a weight percentage basis, 6.67% fosinopril sodium, 5% chlorthalidone, 81.58% lactose, 2.25% povidine, 3.5% crospovidone, and 1% sodium stearyl fumarate.

**8.** The tablet of Claim 5 containing, on a weight percentage basis, 5% fosinopril sodium, 7.5% chlorthalidone, 80.5% lactose, 2.25% povidone, 3.75% crospovidone, and 1% sodium stearyl fumarate.

**9.** The tablet of Claim 5 containing, on a weight percentage basis, 5% fosinopril sodium, 25% chlorthalidone, 62.5% lactose, 3.0% povidone, 3.5% crospovidone, and 1% sodium stearyl fumarate.

**10.** The tablet of Claim 5 containing, on a weight percentage basis, 5% fosinopril sodium, 5% chlorthalidone, 82.5% lactose, 3% povidone, 3.5% crospovidone, and 1% sodium stearyl fumarate.

**11.** The tablet of Claim 3 wherein the only active ingredient is fosinopril sodium.

**12.** The tablet of Claim 11 wherein said filler is a mixture of lactose and microcrystalline cellulose, said disintegrant is crospovidone, and said binder is povidone.

**13.** The tablet of Claim 12 containing, on a weight percentage basis, 2.5% fosinopril sodium, 69.75% lactose, 20% microcrystalline cellulose, 3.5% crospovidone, 2.25% povidone, and 2% sodium stearyl fumarate.

**14.** The tablet of Claim 12 containing, on a weight percentage basis, 5% fosinopril sodium, 67.25% lactose, 20% microcrystalline cellulose, 3.5% crospovidone, 2.25% povidone, and 2% sodium stearyl fumarate.

**15.** The tablet of Claim 12 containing, on a weight percentage basis, 10% fosinopril sodium, 62.25% lactose, 20% microcrystalline cellulose, 3.5% crospovidone, 2.25% povidone, and 2% sodium stearyl fumarate.

**16.** The tablet of Claim 12 containing, on a weight percentage basis, 2.5% fosinopril sodium, 71.5% lactose, 20% microcrystalline cellulose, 3.5% crospovidone, 2% povidone, and 0.5% sodium stearyl fumarate.

**17.** The tablet of Claim 3 containing from 1% to 25% by weight of hydrochlorothiazide.

**18.** The tablet of Claim 17 wherein said filler is lactose or a blend of lactose and microcrystalline cellulose, said disintegrant is cross linked sodium carboxymethylcellulose or sodium starch glycolate, said binder is povidone, and said single agent which is both binder and disintegrant is pregelatinized starch.

**19.** The tablet of Claim 18 containing, on a weight percentage basis, 6.6% fosinopril sodium, 4.2% hydrochlorothiazide, 79.2% lactose, 2.0% povidone, 5.0% cross linked sodium carboxymethylcellulose, and 3.0% sodium stearyl fumarate.

**20.** The tablet of Claim 18 containing, on a weight percentage basis, 5.0% fosinopril sodium, 6.25% hydrochlorothiazide, 78.75% lactose, 2.0% povidone, 5.0% cross linked sodium carboxymethylcellulose, and 3.0% sodium stearyl fumarate.

**21.** The tablet of Claim 18 containing, on a weight percentage basis, 10.0% fosinopril sodium, 6.25% hydrochlorothiazide, 56.25% lactose, 20.0% microcrystalline cellulose, 2.0% povidone, 3.5% sodium starch glycolate, and 2.0% sodium stearyl fumarate.

**22.** The tablet of Claim 18 containing, on a weight percentage basis, 5.0% fosinopril sodium, 6.25% hydrochlorothiazide, 61.75% lactose, 20.0% microcrystalline cellulose, 2.0% povidone, 3.5% sodium starch glycolate, and 1.5% sodium stearyl fumarate.

**23.** The tablet of Claim 18 containing, on a weight percentage basis, 10.0% fosinopril sodium, 6.25% hydrochlorothiazide, 53.75% lactose, 20.0% microcrystalline cellulose, 8.0% pregelatinized starch, and 2.0% sodium stearyl fumarate.

**24.** The tablet of Claim 18 containing, on a weight percentage basis, 5.0% fosinopril sodium, 6.25% hydrochlorothiazide, 59.25% lactose, 20.0% microcrystalline cellulose, 8.0% pregelatinized starch, and 1.5% sodium stearyl fumarate.

**25.** A method of making the tablet of any preceding claim comprising formulating a composition capable of making a tablet having the indicated ingredients in the indicated proportions and forming a tablet from the composition.

**Claims for the following Contracting States : ES, GR**

**1.** A method of making a tablet with improved storage stability comprising, on a weight percentage basis, from 1% to 25% fosinopril sodium, from 0% to 25% of a diuretic, from 30% to 90% of a filler, from 2% to 10% of a disintegrant, and from 1% to 5% of a binder, or from 5% to 15% of a single agent which is both binder and disintegrant, said method comprising:
(a) providing said ingredients;
(b) including in said ingredients from 0.3% to 4% of a lubricant selected from sodium stearyl fumarate and hydrogenated vegetable oil; and
(c) forming a tablet containing said ingredients and said lubricant.

**2.** The method of claim 1 wherein said lubricant is sodium stearyl fumarate.

**3.** The method of claim 2 wherein said filler is lactose or a blend of lactose and microcrystalline cellulose, said disintegrant is selected from sodium carboxymethyl starch, cross linked sodium carboxymethyl starch, crospovidone, cross linked sodium carboxymethylcellulose, sodium starch glycolate, and mixtures thereof, said binder is selected from povidone, hydroxypropyl cellulose, and mixtures thereof, and said single agent which is both binder and disintegrant is pregelatinized starch.

**4.** The method of claim 3 wherein said tablet contains from 1% to 25% by weight of chlorthalidone.

**5.** The method of claim 4 wherein said filler is lactose, said disintegrant is crospovidone, and said binder is povidone.

**6.** The method of claim 5 wherein said tablet contains, on a weight percentage basis, 10% fosinopril sodium, 3.75% chlorthalidone, 79.5% lactose, 2.25% povidone, 3.5% crospovidone, and 1% sodium stearyl fumarate.

**7.** The method of claim 5 wherein said tablet contains, on a weight percentage basis, 6.67% fosinopril sodium, 5% chlorthalidone, 81.58% lactose, 2.25% povidone, 3.5% crospovidone, and 1% sodium stearyl fumarate.

**8.** The method of claim 5 wherein said tablet contains, on a weight percentage basis, 5% fosinopril sodium, 7.5% chlorthalidone, 80.5% lactose, 2.25% povidone, 3.75% crospovidone, and 1% sodium stearyl fumarate.

**9.** The method of claim 5 wherein said tablet comprises, on a weight percentage basis, 5% fosinopril sodium, 25% chlorthalidone, 62.5% lactose, 3.0% povidone, 3.5% crospovidone, and 1% sodium stearyl fumarate.

**10.** The method of claim 5 wherein said tablet comprises, on a weight percentage basis, 5% fosinopril sodium, 5% chlorthalidone, 82.5% lactose, 3% povidone, 3.5% crospovidone, and 1% sodium stearyl fumarate.

**11.** The method of claim 3 wherein the only active ingredient is fosinopril sodium.

**12.** The method of claim 11 wherein said filler is a mixture of lactose and microcrystalline cellulose, said disintegrant is crospovidone, and said binder is povidone.

13. The method of claim 12 wherein said tablet contains, on a weight percentage basis, 2.5% fosinopril sodium, 69.7% lactose, 20% microcrystalline cellulose, 3.5% crospovidone, 2.25% povidone, and 2% sodium stearyl fumarate.

14. The method of claim 12 wherein said tablet contains, on a weight percentage basis, 5% fosinopril sodium, 67.25% lactose, 20% microcrystalline cellulose, 3.5% crospovidone, 2.25% povidone, and 2% sodium stearyl fumarate.

15. The method of claim 12 wherein said tablet contains, on a weight percentage basis, 10% fosinopril sodium, 62.25% lactose, 20% microcrystalline cellulose, 3.5% crospovidone, 2.25% povidone, and 2% sodium stearyl fumarate.

16. The method of claim 12 wherein said tablet contains, on a weight percentage basis, 2.5% fosinopril sodium, 71.5% lactose, 20% microcrystalline cellulose, 3.5% crospovidone, 2% povidone, and 0.5% sodium stearyl fumarate.

17. The method of claim 3 wherein said tablet contains from 1% to 25% by weight of hydrochlorothiazide.

18. The method of claim 17 wherein said filler is lactose or a blend of lactose and microcrystalline cellulose, said disintegrant is cross linked sodium carboxymethylcellulose or sodium starch glycolate, said binder is povidone, and said single agent which is both binder and disintegrant is pregelatinized starch.

19. The method of claim 18 wherein said tablet contains, on a weight percentage basis, 6.6% fosinopril sodium, 4.2% hydrochlorothiazide, 79.2% lactose, 2.0% povidone, 5.0% cross linked sodium carboxymethylcellulose, and 3.0% sodium stearyl fumarate.

20. The method of claim 18 wherein said tablet contains, on a weight percentage basis, 5.0% fosinopril sodium, 6.25% hydrochlorothiazide, 78.75% lactose, 2.0% povidone, 5.0% cross linked sodium carboxymethylcellulose, and 3.0% sodium stearyl fumarate.

21. The method of claim 18 wherein said tablet contains, on a weight percentage basis, 10.0% fosinopril sodium, 6.25% hydrochlorothiazide, 56.25% lactose, 20.0% microcrystalline cellulose, 2.0% povidone, 3.5% sodium starch glycolate, and 2.0% sodium stearyl fumarate.

22. The method of claim 18 wherein said tablet contains, on a weight percentage basis, 5.0% fosinopril sodium, 6.25% hydrochlorothiazide, 61.75% lactose, 20.0% microcrystalline cellulose, 2.0% povidone, 3.5% sodium starch glycolate, and 1.5% sodium stearyl fumarate.

23. The method of claim 18 wherein said tablet contains, on a weight percentage basis, 10.0% fosinopril sodium, 6.25% hydrochlorothiazide, 53.75% lactose, 20.0% microcrystalline cellulose, 8.0% pregelatinized starch, and 2.0% sodium stearyl fumarate.

24. The method of claim 18 wherein said tablet contains, on a weight percentage basis, 5.0% fosinopril sodium, 6.25% hydrochlorothiazide, 59.25% lactose, 20.0% microcrystalline cellulose, 8.0% pregelatinized starch, and 1.5% sodium stearyl fumarate.

25. The method of any preceding claim which comprises wet granulation or dry granulation.

## Patentansprüche

### Patentansprüche für folgende Vertragsstaaten : AT, BE, CH, DE, DK, FR, GB, IT, LI, LU, NL, SE

1. Stabile Tablette, die auf Gewichtsprozentbasis 1 bis 25% Fosinopril-Natrium, 0 bis 25% eines Diuretikums, 30 bis 90% eines Füllstoffs, 2 bis 10% eines Sprengmittels, 1 bis 5% eines Bindemittels oder 5 bis 15% eines einzelnen Mittels, das sowohl Binde- als auch Sprengmittel ist, und 0,3 bis 4% eines aus Natriumstearylfumarat und hydriertem Pflanzenöl ausgewählten Schmierstoffs umfaßt.

2. Tablette nach Anspruch 1, wobei der Schmierstoff Natriumstearylfumarat ist.

3. Tablette nach Anspruch 2, wobei der Füllstoff Lactose oder ein Gemisch aus Lactose und mikrokristalliner Cellulose ist, das Sprengmittel aus Natriumcarboxymethylstärke, vernetzter Natriumcarboxymethylstärke, Crospovidon, vernetzter Natriumcarboxymethylcellulose, Natriumstärkeglykolat und Gemischen davon ausgewählt ist, das Bindemittel aus Povidon, Hydroxypropylcellulose und Gemischen davon ausgewählt ist und das einzelne Mittel, das sowohl Binde- als auch Sprengmittel ist, vorgelierte Stärke ist.

4. Tablette nach Anspruch 3, die 1 bis 25 Gew.-% Chlortalidon enthält.

5. Tablette nach Anspruch 4, wobei der Füllstoff Lactose ist, das Sprengmittel Crospovidon ist und das Bindemittel Povidon ist.

6. Tablette nach Anspruch 5, die auf Gewichtsprozentbasis 10% Fosinopril-Natrium, 3,75% Chlortalidon, 79,5% Lactose, 2,25% Povidon, 3,5% Crospovidon und 1% Natriumstearylfumarat enthält.

7. Tablette nach Anspruch 5, die auf Gewichtsprozentbasis 6,67% Fosinopril-Natrium, 5% Chlortalidon, 81,58% Lactose, 2,25% Povidon, 3,5% Crospovidon und 1% Natriumstearylfumarat enthält.

8. Tablette nach Anspruch 5, die auf Gewichtsprozentbasis 5% Fosinopril-Natrium, 7,5% Chlortalidon, 80,5% Lactose, 2,25% Povidon, 3,75% Crospovidon und 1% Natriumstearylfumarat enthält.

9. Tablette nach Anspruch 5, die auf Gewichtsprozentbasis 5% Fosinopril-Natrium, 25% Chlortalidon, 62,5% Lactose, 3,0% Povidon, 3,5% Crospovidon und 1% Natriumstearylfumarat enthält.

10. Tablette nach Anspruch 5, die auf Gewichtsprozentbasis 5% Fosinopril-Natrium, 5% Chlortalidon, 82,5% Lactose, 3% Povidon, 3,5% Crospovidon und 1% Natriumstearylfumarat enthält.

11. Tablette nach Anspruch 3, in der der einzige Wirkstoff Fosinopril-Natrium ist.

12. Tablette nach Anspruch 11, wobei der Füllstoff ein Gemisch aus Lactose und mikrokristalliner Cellulose ist, das Sprengmittel Crospovidon ist und das Bindemittel Povidon ist.

13. Tablette nach Anspruch 12, die auf Gewichtsprozentbasis 2,5% Fosinopril-Natrium, 69,75% Lactose, 20% mikrokristalline Cellulose, 3,5% Crospovidon, 2,25% Povidon und 2% Natriumstearylfumarat enthält.

14. Tablette nach Anspruch 12, die auf Gewichtsprozentbasis 5% Fosinopril-Natrium, 67,25% Lactose, 20% mikrokristalline Cellulose, 3,5% Crospovidon, 2,25% Povidon und 2% Natriumstearylfumarat enthält.

15. Tablette nach Anspruch 12, die auf Gewichtsprozentbasis 10% Fosinopril-Natrium, 62,25% Lactose, 20% mikrokristalline Cellulose, 3,5% Crospovidon, 2,25% Povidon und 2% Natriumstearylfumarat enthält.

16. Tablette nach Anspruch 12, die auf Gewichtsprozentbasis, 2,5% Fosinopril-Natrium, 71,5% Lactose, 20% mikrokristalline Cellulose, 3,5% Crospovidon, 2% Povidon und 0,5% Natriumstearylfumarat enthält.

17. Tablette nach Anspruch 3, die 1 bis 25 Gew.-% Hydrochlorothiazid enthält.

18. Tablette nach Anspruch 17, wobei der Füllstoff Lactose oder ein Gemisch aus Lactose und mikrokristalliner Cellulose ist, das Sprengmittel vernetzte Natriumcarboxymethylcellulose oder Natriumstärkeglykolat ist, das Bindemittel Povidon ist und das einzelne Mittel, das sowohl Binde- als auch Sprengmittel ist, vorgelierte Stärke ist.

19. Tablette nach Anspruch 18, die auf Gewichtsprozentbasis 6,6% Fosinopril-Natrium, 4,2% Hydrochlorothiazid, 79,2% Lactose, 2,0% Povidon, 5,0% vernetzte Natriumcarboxymethylcellulose und 3,0% Natriumstearylfumarat enthält.

20. Tablette nach Anspruch 18, die auf Gewichtsprozentbasis 5,0% Fosinopril-Natrium, 6,25% Hydrochlorothiazid, 78,75% Lactose, 2,0% Povidon, 5,0% vernetzte Natriumcarboxymethylcellulose und 3,0% Natriumstearylfumarat enthält.

21. Tablette nach Anspruch 18, die auf Gewichtsprozentbasis 10,0% Fosinopril-Natrium, 6,25% Hydrochlorothiazid, 56,25% Lactose, 20,0% mikrokristalline Cellulose, 2,0% Povidon, 3,5% Natriumstärkeglykolat und 2,0% Natriumstearylfumarat enthält.

22. Tablette nach Anspruch 18, die auf Gewichtsprozentbasis, 5,0% Fosinopril-Natrium, 6,25% Hydrochlorothiazid, 61,75% Lactose, 20,0% mikrokristalline Cellulose, 2,0% Povidon, 3,5% Natriumstärkeglykolat und 1,5% Natriumstearylfumarat enthält.

23. Tablette nach Anspruch 18, die auf Gewichtsprozentbasis 10,0% Fosinopril-Natrium, 6,25% Hydrochlorothiazid, 53,75% Lactose, 20,0% mikrokristalline Cellulose, 8,0% vorgelierte Stärke und 2,0% Natriumstearylfumarat enthält.

24. Tablette nach Anspruch 18, die auf Gewichtsprozentbasis 5,0% Fosinopril-Natrium, 6,25% Hydrochlorothiazid, 59,25% Lactose, 20,0% mikrokristalline Cellulose, 8,0% vorgelierte Stärke und 1,5% Natriumstearylfumarat enthält.

25. Verfahren zur Herstellung der Tablette eines vorstehenden Anspruchs, das das Formulieren einer Zusammensetzung, womit sich eine Tablette mit den angegebenen Bestandteilen in den angegebenen Verhältnissen herstellen läßt, und das das Formen einer Tablette aus der Zusammensetzung umfaßt.

**Patentansprüche für folgende Vertragsstaaten : ES, GR**

1. Verfahren zur Herstellung einer Tablette mit verbesserter Lagerungsstabilität, die auf Gewichtsprozentbasis 1 bis 25% Fosinopril-Natrium, 0 bis 25% eines Diuretikums, 30 bis 90% eines Füllstoffs, 2 bis 10% eines Sprengmittels und 1 bis 5% eines Bindemittels oder 5 bis 15% eines einzelnen Mittels, das sowohl Binde- als auch Sprengmittel ist, umfaßt, wobei das Verfahren:
   (a) Bereitstellen der Bestandteile;
   (b) Einschließen von 0,3 bis 4% eines aus Natriumstearylfumarat und hydriertem Pflanzenöl ausgewählten Schmierstoffs in die Bestandteile und
   (c) Formen einer Tablette, die die Bestandteile und den Schmierstoff enthält,
   umfaßt.

2. Verfahren nach Anspruch 1, wobei der Schmierstoff Natriumstearylfumarat ist.

3. Verfahren nach Anspruch 2, wobei der Füllstoff Lactose oder ein Gemisch aus Lactose und mikrokristalliner Cellulose ist, das Sprengmittel aus Natriumcarboxymethylstärke, vernetzter Natriumcarboxymethylstärke, Crospovidon, vernetzter Natriumcarboxymethylcellulose, Natriumstärkeglykolat und Gemischen davon ausgewählt ist, das Bindemittel aus Povidon, Hydroxypropylcellulose und Gemischen davon ausgewählt ist und das einzelne Mittel, das sowohl Binde- als auch Sprengmittel ist, vorgelierte Stärke ist.

4. Verfahren nach Anspruch 3, wobei die Tablette 1 bis 25 Gew.-% Chlortalidon enthält.

5. Verfahren nach Anspruch 4, wobei der Füllstoff Lactose ist, das Sprengmittel Crospovidon ist und das Bindemittel Povidon ist.

6. Verfahren nach Anspruch 5, wobei die Tablette auf Gewichtsprozentbasis 10% Fosinopril-Natrium, 3,75% Chlortalidon, 79,5% Lactose, 2,25% Povidon, 3,5% Crospovidon und 1% Natriumstearylfumarat enthält.

7. Verfahren nach Anspruch 5, wobei die Tablette auf Gewichtsprozentbasis 6,67% Fosinopril-Natrium, 5% Chlortalidon, 81,58% Lactose, 2,25% Povidon, 3,5% Crospovidon und 1% Natriumstearylfumarat enthält.

8. Verfahren nach Anspruch 5, wobei die Tablette auf Gewichtsprozentbasis 5% Fosinopril-Natrium, 7,5% Chlortalidon, 80,5% Lactose, 2,25% Povidon, 3,75% Crospovidon und 1% Natriumstearylfumarat enthält.

9. Verfahren nach Anspruch 5, wobei die Tablette auf Gewichtsprozentbasis 5% Fosinopril-Natrium, 25% Chlortalidon, 62,5% Lactose, 3,0% Povidon, 3,5% Crospovidon und 1% Natriumstearylfumarat umfaßt.

10. Verfahren nach Anspruch 5, wobei die Tablette auf Gewichtsprozentbasis 5% Fosinopril-Natrium, 5% Chlortalidon, 82,5% Lactose, 3% Povidon, 3,5% Crospovidon und 1% Natriumstearylfumarat umfaßt.

11. Verfahren nach Anspruch 3, in der der einzige Wirkstoff Fosinopril-Natrium ist.

12. Verfahren nach Anspruch 11, wobei der Füllstoff ein Gemisch aus Lactose und mikrokristalliner Cellulose ist, das Sprengmittel Crospovidon ist und das Bindemittel Povidon ist.

13. Verfahren nach Anspruch 12, wobei die Tablette auf Gewichtsprozentbasis 2,5% Fosinopril-Natrium, 69,7% Lactose, 20% mikrokristalline Cellulose, 3,5% Crospovidon, 2,25% Povidon und 2% Natriumstearylfumarat enthält.

14. Verfahren nach Anspruch 12, wobei die Tablette auf Gewichtsprozentbasis, 5% Fosinopril-Natrium, 67,25% Lactose, 20% mikrokristalline Cellulose, 3,5% Crospovidon, 2,25% Povidon und 2% Natriumstearylfumarat enthält.

15. Verfahren nach Anspruch 12, wobei die Tablette auf Gewichtsprozentbasis 10% Fosinopril-Natrium, 62,25% Lactose, 20% mikrokristalline Cellulose, 3,5% Crospovidon, 2,25% Povidon und 2% Natriumstearylfumarat enthält.

16. Verfahren nach Anspruch 12, wobei die Tablette auf Gewichtsprozentbasis, 2,5% Fosinopril-Natrium, 71,5% Lactose, 20% mikrokristalline Cellulose, 3,5% Crospovidon, 2% Povidon und 0,5% Natriumstearylfumarat enthält.

17. Verfahren nach Anspruch 3, wobei die Tablette 1 bis 25 Gew.-% Hydrochlorothiazid enthält.

18. Verfahren nach Anspruch 17, wobei der Füllstoff Lactose oder ein Gemisch aus Lactose und mikrokristalliner Cellulose ist, das Sprengmittel vernetzte Natriumcarboxymethylcellulose oder Natriumstärkeglykolat ist, das Bindemittel Povidon ist und das einzelne Mittel, das sowohl Binde- als auch Sprengmittel ist, vorgelierte Stärke ist.

19. Verfahren nach Anspruch 18, wobei die Tablette auf Gewichtsprozentbasis, 6,6% Fosinopril-Natrium, 4,2% Hydrochlorothiazid, 79,2% Lactose, 2,0% Povidon, 5,0% vernetzte Natriumcarboxymethylcellulose und 3,0% Natriumstearylfumarat enthält.

20. Verfahren nach Anspruch 18, wobei die Tablette auf Gewichtsprozentbasis 5,0% Fosinopril-Natrium, 6,25% Hydrochlorothiazid, 78,75% Lactose, 2,0% Povidon, 5,0% vernetzte Natriumcarboxymethylcellulose und 3,0% Natriumstearylfumarat enthält.

21. Verfahren nach Anspruch 18, wobei die Tablette auf Gewichtsprozentbasis 10,0% Fosinopril-Natrium, 6,25% Hydrochlorothiazid, 56,25% Lactose, 20,0% mikrokristalline Cellulose, 2,0% Povidon, 3,5% Natriumstärkeglykolat und 2,0% Natriumstearylfumarat enthält.

22. Verfahren nach Anspruch 18, wobei die Tablette auf Gewichtsprozentbasis 5,0% Fosinopril-Natrium, 6,25% Hydrochlorothiazid, 61,75% Lactose, 20,0% mikrokristalline Cellulose, 2,0% Povidon, 3,5% Natriumstärkeglykolat und 1,5% Natriumstearylfumarat enthält.

23. Verfahren nach Anspruch 18, wobei die Tablette auf Gewichtsprozentbasis 10,0% Fosinopril-Natrium, 6,25% Hydrochlorothiazid, 53,75% Lactose, 20,0% mikrokristalline Cellulose, 8,0% vorgelierte Stärke und 2,0% Natriumstearylfumarat enthält.

24. Verfahren nach Anspruch 18, wobei die Tablette auf Gewichtsprozentbasis, 5,0% Fosinopril-Natrium, 6,25% Hydrochlorothiazid, 59,25% Lactose, 20,0% mikrokristalline Cellulose, 8,0% vorgelierte Stärke und 1,5% Natriumstearylfumarat enthält.

25. Verfahren nach einem der vorstehenden Ansprüche, das Naßgranulation oder Trockengranulation umfaßt.

## Revendications

### Revendications pour les Etats contractants suivants : AT, BE, CH, DE, DK, FR, GB, IT, LI, LU, NL, SE

1. Comprimé stable comprenant, en pourcentages pondéraux, de 1% à 25% de fosinopril sodique, de 0% à 25% d'un diurétique, de 30% à 90% d'une charge, de 2% à 10% d'un agent de désintégration, de 1% à 5% d'un liant, ou bien de 5% à 15% d'un agent unique qui sert à la fois de liant et d'agent de désintégration, et de 0,3% à 4% d'un lubrifiant choisi entre le stéaryl fumarate de sodium et une huile végétale hydrogénée.

2. Comprimé selon la revendication 1, dans lequel ledit lubrifiant est le stéaryl fumarate de sodium.

3. Comprimé selon la revendication 2, dans lequel ladite charge est le lactose ou un mélange de lactose et de cellulose microcristalline, ledit agent de désintégration est choisi entre le carboxyméthyl amidon sodique, le carboxyméthyl amidon sodique réticulé, la crospovidone, la carboxyméthylcellulose sodique réticulée, le glycolate d'amidon sodique, et leurs mélanges, ledit liant est choisi entre la povidone, l'hydroxypropyl cellulose et leurs mélanges, et ledit agent unique servant à la fois de liant et d'agent de désintégration est l'amidon prégélatinisé.

4. Comprimé selon la revendication 3, contenant de 1% à 25% en poids de chlortalidone.

5. Comprimé selon la revendication 4, dans lequel ladite charge est le lactose, ledit agent de désintégration est la crospovidone, et ledit liant est la povidone.

6. Comprimé selon la revendication 5, contenant, en pourcentages pondéraux, 10% de fosinopril sodique, 3,75% de chlortalidone, 79,5% de lactose, 2,25% de povidone, 3,5% de crospovidone, et 1% de stéaryl fumarate de sodium.

7. Comprimé selon la revendication 5, contenant, en pourcentages pondéraux, 6,67% de fosinopril sodique, 5% de chlortalidone, 81,58% de lactose, 2,25% de povidone, 3,5% de crospovidone, et 1% de stéaryl fumarate de sodium.

8. Comprimé selon la revendication 5, contenant, en pourcentages pondéraux, 5% de fosinopril sodique, 7,5% de chlortalidone, 80,5% de lactose, 2,25% de povidone, 3,75% de crospovidone, et 1% de stéaryl fumarate de sodium.

9. Comprimé selon la revendication 5, contenant, en pourcentages pondéraux, 5% de fosinopril sodique, 25% de chlortalidone, 62,5% de lactose, 3,0% de povidone, 3,5% de crospovidone, et 1% de stéaryl fumarate de sodium.

10. Comprimé selon la revendication 5, contenant, en pourcentages pondéraux, 5% de fosinopril sodique, 5% de chlortalidone, 82,5% de lactose, 3% de povidone, 3,5% de crospovidone, et 1% de stéaryl fumarate de sodium.

11. Comprimé selon la revendication 3, dans lequel le seul ingrédient actif est le fosinopril sodique.

12. Comprimé selon la revendication 11, dans lequel ladite charge est un mélange de lactose et de cellulose microcristalline, ledit agent de désintégration est la crospovidone, et ledit liant est la povidone.

13. Comprimé selon la revendication 12, contenant, en pourcentages pondéraux, 2,5% de fosinopril sodique, 69,75% de lactose, 20% de cellulose microcristalline, 3,5% de crospovidone, 2,25% de povidone, et 2% de stéaryl fumarate de sodium.

14. Comprimé selon la revendication 12, contenant, en pourcentages pondéraux, 5% de fosinopril sodique, 67,25% de lactose, 20% de cellulose microcristalline, 3,5% de crospovidone, 2,25% de povidone, et 2% de stéaryl fumarate de sodium.

15. Comprimé selon la revendication 12, contenant, en pourcentages pondéraux, 10% de fosinopril sodique, 62,25% de lactose, 20% de cellulose microcristalline, 3,5% de crospovidone, 2,25% de povidone, et 2%

de stéaryl fumarate de sodium.

16. Comprimé selon la revendication 12, contenant, en pourcentages pondéraux, 2,5% de fosinopril sodique, 71,5% de lactose, 20% de cellulose microcristalline, 3,5% de crospovidone, 2% de povidone, et 0,5% de stéaryl fumarate de sodium.

17. Comprimé selon la revendication 3, contenant de 1% à 25% en poids d'hydrochlorothiazide.

18. Comprimé selon la revendication 17, dans lequel ladite charge est le lactose ou un mélange de lactose et de cellulose microcristalline, ledit agent de désintégration est la carboxyméthylcellulose sodique réticulée ou le glycolate d'amidon sodique, ledit liant est la povidone, et ledit agent unique qui sert à la fois de liant et d'agent de désintégration est l'amidon prégélatinisé .

19. Comprimé selon la revendication 18, contenant, en pourcentages pondéraux, 6,6% de fosinopril sodique, 4,2% d'hydrochlorothiazide, 79,2% de lactose, 2,0% de povidone, 5,0% de carboxyméthylcellulose sodique réticulée, et 3,0% de stéaryl fumarate de sodium.

20. Comprimé selon la revendication 18, contenant, en pourcentages pondéraux, 5,0% de fosinopril sodique, 6,25% d'hydrochlorothiazide, 78,75% de lactose, 2,0% de povidone, 5,0% de carboxyméthylcellulose sodique réticulée, et 3,0% de stéaryl fumarate de sodium.

21. Comprimé selon la revendication 18, contenant, en pourcentages pondéraux, 10,0% de fosinopril sodique, 6,25% d'hydrochlorothiazide, 56,25% de lactose, 20,0% de cellulose microcristalline, 2,0% de povidone, 3,5% de glycolate d'amidon sodique, et 2,0% de stéaryl fumarate de sodium.

22. Comprimé selon la revendication 18, contenant, en pourcentages pondéraux, 5,0% de fosinopril sodique, 6,25% d'hydrochlorothiazide, 61,75% de lactose, 20,0% de cellulose microcristalline, 2,0% de povidone, 3,5% de glycolate d'amidon sodique, et 1,5% de stéaryl fumarate de sodium.

23. Comprimé selon la revendication 18, contenant, en pourcentages pondéraux, 10,0% de fosinopril sodique, 6,25% d'hydrochlorothiazide, 53,75% de lactose, 20,0% de cellulose microcristalline, 8,0% d'amidon prégélatinisé, et 2,0% de stéaryl fumarate de sodium.

24. Comprimé selon la revendication 18, contenant, en pourcentages pondéraux, 5,0 % de fosinopril sodique, 6,25% d'hydrochlorothiazide, 59,25% de lactose, 20,0% de cellulose microcristalline, 8,0% d'amidon prégélatinisé, et 1,5% de stéaryl fumarate de sodium.

25. Procédé de fabrication du comprimé selon l'une quelconque des revendications précédentes, consistant à formuler une composition capable de former un comprimé contenant les ingrédients indiqués dans les proportions indiquées, et à former un comprimé à partir de cette composition.

**Revendications pour les Etats contractants suivants : ES, GR**

1. Procédé de fabrication d'un comprimé à stabilité d'entreposage améliorée, comprenant, en pourcentages pondéraux, de 1% à 25% de fosinopril sodique, de 0% à 25% d'un diurétique, de 30% à 90% d'une charge, de 2% à 10% d'un agent de désintégration, et de 1% à 5% d'un liant, ou bien de 5% à 15% d'un agent unique qui sert à la fois de liant et d'agent de désintégration, ledit procédé consistant:
   (a) à prendre lesdits ingrédients;
   (b) à ajouter auxdits ingrédients de 0,3% à 4% d'un lubrifiant choisi entre le stéaryl fumarate de sodium et une huile végétale hydrogénée; et
   (c) à former un comprimé contenant lesdits ingrédients et ledit lubrifiant.

2. Procédé selon la revendication 1, dans lequel ledit lubrifiant est le stéaryl fumarate de sodium.

3. Procédé selon la revendication 2, dans lequel ladite charge est le lactose ou un mélange de lactose et de cellulose microcristalline, ledit agent de désintégration est choisi entre le carboxyméthyl amidon sodique, le carboxyméthyl amidon sodique réticulé, la crospovidone, la carboxyméthylcellulose sodique réticulée, le glycolate d'amidon sodique, et leurs mélanges, ledit liant est choisi entre la povidone, l'hydroxypropyl cellulose et leurs mélanges, et ledit agent unique servant à la fois de liant et d'agent de désintégration est l'amidon prégélatinisé.

4. Procédé selon la revendication 3, dans lequel ledit comprimé contient de 1% à 25% en poids de chlortalidone.

5. Procédé selon la revendication 4, dans lequel ladite charge est le lactose, ledit agent de désintégration est la crospovidone, et ledit liant est la povidone.

6. Procédé selon la revendication 5, dans lequel ledit comprimé contient, en pourcentages pondéraux, 10% de fosinopril sodique, 3,75% de chlortalidone, 79,5% de lactose, 2,25% de povidone, 3,5% de crospovidone, et 1% de stéaryl fumarate de sodium.

7. Procédé selon la revendication 5, dans lequel ledit comprimé contient, en pourcentages pondéraux, 6,67% de fosinopril sodique, 5% de chlortalidone, 81,58% de lactose, 2,25% de povidone, 3,5% de crospovidone, et 1% de stéaryl fumarate de sodium.

8. Procédé selon la revendication 5, dans lequel ledit comprimé contient, en pourcentages pondéraux, 5% de fosinopril sodique, 7,5% de chlortalidone, 80,5% de lactose, 2,25% de povidone, 3,75% de crospovidone, et 1% de stéaryl fumarate de sodium.

9. Procédé selon la revendication 5, dans lequel ledit comprimé contient, en pourcentages pondéraux, 5% de fosinopril sodique, 25% de chlortalidone, 62,5% de lactose, 3,0% de povidone, 3,5% de crospovidone, et 1% de stéaryl fumarate de sodium.

10. Procédé selon la revendication 5, dans lequel ledit comprimé contient, en pourcentages pondéraux, 5% de fosinopril sodique, 5% de chlortalidone, 82,5% de lactose, 3% de povidone, 3,5% de crospovidone, et 1% de stéaryl fumarate de sodium.

11. Procédé selon la revendication 3, dans lequel le seul ingrédient actif est le fosinopril sodique.

12. Procédé selon la revendication 11, dans lequel ladite charge est un mélange de lactose et de cellulose microcristalline, ledit agent de désintégration est la crospovidone, et ledit liant est la povidone.

13. Procédé selon la revendication 12, dans lequel ledit comprimé contient, en pourcentages pondéraux, 2,5% de fosinopril sodique, 69,7 % de lactose, 20% de cellulose microcristalline, 3,5% de crospovidone, 2,25% de povidone, et 2% de stéaryl fumarate de sodium.

14. Procédé selon la revendication 12, dans lequel ledit comprimé contient, en pourcentages pondéraux, 5% de fosinopril sodique, 67,25% de lactose, 20% de cellulose microcristalline, 3,5% de crospovidone, 2,25% de povidone, et 2% de stéaryl fumarate de sodium.

15. Procédé selon la revendication 12, dans lequel ledit comprimé contient, en pourcentages pondéraux, 10% de fosinopril sodique, 62,25% de lactose, 20% de cellulose microcristalline, 3,5% de crospovidone, 2,25% de povidone, et 2% de stéaryl fumarate de sodium.

16. Procédé selon la revendication 12, dans lequel ledit comprimé contient, en pourcentages pondéraux, 2,5% de fosinopril sodique, 71,5% de lactose, 20% de cellulose microcristalline, 3,5% de crospovidone, 2% de povidone, et 0,5% de stéaryl fumarate de sodium.

17. Procédé selon la revendication 3, dans lequel ledit comprimé contient de 1% à 25% en poids d'hydrochlorothiazide.

18. Procédé selon la revendication 17, dans lequel ladite charge est le lactose ou un mélange de lactose et de cellulose microcristalline, ledit agent de désintégration est la carboxyméthylcellulose sodique réticulée ou le glycolate d'amidon sodique, ledit liant est la povidone, et ledit agent unique qui sert à la fois de liant et d'agent de désintégration est l'amidon prégélatinisé .

19. Procédé selon la revendication 18, dans lequel ledit comprimé contient, en pourcentages pondéraux, 6,6% de fosinopril sodique, 4,2% d'hydrochlorothiazide, 79,2% de lactose, 2,0% de povidone, 5,0% de carboxyméthylcellulose sodique réticulée, et 3,0% de stéaryl fumarate de sodium.

20. Procédé selon la revendication 18, dans lequel ledit comprimé contient, en pourcentages pondéraux, 5,0%

de fosinopril sodique, 6,25% d'hydrochlorothiazide, 78,75% de lactose, 2,0% de povidone, 5,0% de carboxyméthylcellulose sodique réticulée, et 3,0% de stéaryl fumarate de sodium.

**21.** Comprimé selon la revendication 18, dans lequel ledit comprimé contient, en pourcentages pondéraux, 10,0% de fosinopril sodique, 6,25% d'hydrochlorothiazide, 56,25% de lactose, 20,0% de cellulose microcristalline, 2,0% de povidone, 3,5% de glycolate d'amidon sodique, et 2,0% de stéaryl fumarate de sodium.

**22.** Procédé selon la revendication 18, dans lequel ledit comprimé contient, en pourcentages pondéraux, 5,0% de fosinopril sodique, 6,25% d'hydrochlorothiazide, 61,75% de lactose, 20,0% de cellulose microcristalline, 2,0% de povidone, 3,5% de glycolate d'amidon sodique, et 1,5% de stéaryl fumarate de sodium.

**23.** Procédé selon la revendication 18, dans lequel ledit comprimé contient, en pourcentages pondéraux, 10,0% de fosinopril sodique, 6,25% d'hydrochlorothiazide, 53,75% de lactose, 20,0% de cellulose microcristalline, 8,0% d'amidon prégélatinisé, et 2,0% de stéaryl fumarate de sodium.

**24.** Procédé selon la revendication 18, dans lequel ledit comprimé contient, en pourcentages pondéraux, 5,0. % de fosinopril sodique, 6,25% d'hydrochlorothiazide, 59,25% de lactose, 20,0% de cellulose microcristalline, 8,0% d'amidon prégélatinisé, et 1,5% de stéaryl fumarate de sodium.

**25.** Procédé selon l'une quelconque des revendications précédentes, qui comprend une granulation à l'état humide ou une granulation à sec.